# EUROPEAN PATENT APPLICATION

(11) **EP 4 389 109 A1**
(43) Date of publication of application: **26.06.2024**
(21) Application number: 22215310.8
(22) Date of filing: 21.12.2022
(51) Int. Cl.: A61K 8/37, A61K 8/04, A61K 8/97, A61K 8/9733, A61K 8/9789, A61Q 15/00

(54) **ECO-SUSTAINABLE COSMETIC COMPOSITION WITH ANTIPERSPIRANT ACTION**

(71) Applicant: Cosmosol Srl, 20121 Milano (IT)
(72) Inventor: NASO, Federica, 26813 Graffignana (LO) (IT); ANELLI, Daniele, 26838 Tavazzano Con Villavesco (IT); SIMONINI, Stefano, 26837 Mulazzano (IT)
(74) Representative: Charrier Rapp & Liebau

(57) **Abstract**

The invention relates to a deodorant cosmetic composition with antiperspirant efficacy, free of aluminum salts and metal compounds, silicones and synthetic filming resins, which comprises, in variable percentages, one or more plant extracts, with astringent activity, Triethyl Citrate and excipients acceptable from the point of view cosmetic. The composition according to the invention allows to obtain an active film-forming and astringent complex, whose activity is based exclusively on natural substances, with an antiperspirant efficacy similar to that attributed to known formulas containing metal compounds, silicones and synthetic filming resins.

## Description

The invention operates in the field of cosmetic products and in particular products with antiperspirant action.

The cosmetic formulations with deodorant and / or antiperspirant activity are intended to remedy the undesirable effects of sweating, physiological function exerted by the eccrine sweat glands, regulating body temperature, and by the apocrine glands, activated by multiple events-stimuli.

Sweat, secreted by the glands through the sweat ducts, is in fact responsible for the formation of unpleasant odors, generated by its interaction with the bacterial flora present on the epidermis.

Products with deodorant-only action are based on formulations that mask the undesirable effect of sweating through a persistent fragrance and generally consist of alcoholic solutions in which an aromatic fragrance, mostly of synthetic origin, is dissolved.

In order to prevent the interaction of sweat with the bacterial flora which, as mentioned, is the cause of the development of bad odors, deodorant products may also contain bacteriostatic substances capable of controlling the proliferation of bacterial flora or broad-spectrum antibacterial agents.

Deodorants that act on the bacterial flora of the skin are generally alcohol-based products, as alcohol, in addition to the function of solubilizing the chosen fragrance, performs a bacteriostatic action.

As a deodorizing and antibacterial agent, it is known in cosmetics, the use of Triethyl Citrate, which has the advantage of being free from sensitization risks.

Unlike deodorants, which only mask the odor effects of sweat already formed or limit its microbial decomposition, antiperspirants (ATP) are cosmetic products that, to prevent the undesirable effects of sweating, act directly on the excretory ducts of the sweat glands, limiting or temporarily blocking the secretion of sweat.

The known antiperspirant deodorants effectively perform their function using certain metal compounds, in particular metal salts.

The effect of antiperspirants based on metal salts against thermal sweating, under normal physiological conditions, has been well studied: they act by blocking the excretions of the sweat glands by precipitating them together with the proteins of the skin, leading to the formation of "plugs" that block or limit the escape of sweat from the hair follicle canal. The metal compounds used as active ingredients to limit or temporarily block the activity of the sweat glands are generally salts of aluminum, magnesium, zinc and zirconium, and preferably aluminum chlorohydrate or aluminum sulphate.

Antiperspirants are commonly presented as silicone reverse emulsions or silicone suspensions that include the active ingredient, such as aluminum chlorohydrate, in varying percentages, additives of various kinds and emulsifiers. Although dry solid solutions are also on the market, containing powdered aluminum salts as an occlusive active, more frequently antiperspirants are marketed in aerosol spray format, as the product, finely vaporized, is distributed more homogeneously and evenly on the skin and is therefore more effective.

GB 2527044, disclose a deodorizing and antiperspirant composition, without components based on aluminum, zirconium and zinc and their salts, in which the antiperspirant action is achieved through the use of a film-forming polymer, consisting of methylvinyl ether and maleic acid residues, and an astringent compound of vegetable origin. In addition, the use of non-aqueous solvents actively contributes to the reduction of sweating of the axillary area.

A first disadvantage of known antiperspirants with aluminum salts, and similar compounds is that, in aerosol spray packaging, they require as propellant the use of flammable gases, potentially harmful to the environment, such as butane, propane, isobutane or LPG and fluorinated propellants such as irdofluorolefins, not allowing instead the use of eco-sustainable spray containers that use compressed gases, such as nitrogen, and equipped with eco-valves as propellants, such as the SSG valve by Salvalco^{®} and the Coster-Eco valve by Coster^{®}, as metal compounds tend to compromise their functioning over time.

In fact, it is known that liquefied gas propellants, having the advantage of maintaining constant pressure inside the can, even when the volume of the product to be dispensed decreases, guarantee an effective and continuous dispersion. Otherwise, the pressure of a compressed gas, such as nitrogen, anhydride or compressed air, decreases as the volume that the gas has available increases, with a significant loss of the delivery force of the can while emptying. In fact, compressed gases such as nitrogen having to subject to the law of ideal gases, pV=nRT.

As shown in the attached graph (source: Compressed Gas Aerosol Insert Design, 25th ILASS - Europe 2013) with the use of compressed gases the pressure decreases considerably during the emptying of aerosol containers with consequent loss of "force" of spraying.

In known formulas containing metal salts, the latter are deposited in the actuator channels and it is impossible to have sufficient force to their dissolution / thrust with propellants such as compressed air, nitrogen or carbon dioxide but it is only possible thanks to hydrocarbon propellants such as butane, propane, isobutane, or fluorinated propellants such as irdofluorolefins.

At the state of the art it is therefore not possible to find antiperspirant products on the market in eco-sustainable spray containers that uses compressed gases as propellants.

A further disadvantage of antiperspirant products, with the use of aluminum salts or similar compounds, consists in the fact that they are responsible for possible phenomena of sensitization or skin irritation. In addition, prolonged use of these compounds could lead to excessive and harmful occlusion of the glandular duct.

At the state of the art in antiperspirant cosmetic formulations there is therefore the need to find compositions free from metal salts such as aluminum, zinc, zirconium and magnesium, and free of silicones, but with an antiperspirant efficacy advantageous or comparable to that attributable to known products.

The invention, therefore, aims to overcome the problems deriving from the possible harmful effects of known formulations, such as toxicity and irritability, with a deodorizing cosmetic formulation with antiperspirant efficacy, but free of aluminum, magnesium, zinc, zirconium or similar compounds.

Another purpose of the invention is to obtain a product that meets the requirements of the so-called clean and natural beauty, that is, cosmetics focused on products that are not only organic and natural, but more generally eco-sustainable, also, for example, with regard to packaging. The purpose of the invention is therefore also to allow the use of natural non-flammable propellants, as an alternative to the well-known hydrocarbon propellants, such as LPG.

The purpose of the invention is also to create a cosmetic, with antiperspirant activity, which uses water or ethanol of vegetable origin as the main solvent and free of cyclic silicones or silicone derivatives used as a solvent, dispersant or cosmetic carrier such as, but not limited to, Cyclomethicone, Cyclopentasiloxane, Cyclohexasiloxane, Trimethicone, Simethicone, Dimethicone, Dimethiconol, Amodimethicone, Dimethiconol, Cetyl Dimethicone, Amodimethicone, Dimethicone copolyol, Trimethysiloxysilicate, Polymethylsilsesquioxane, Polypropylsilsesquioxane.

Finally, the invention aims to create an antiperspirant deodorant free of paraben preservatives or synthetic preservatives and preferably with a eudermic pH between 4.5 and 5.5.

It has been found, and is the subject of the present invention, that Triethyl Citrate, leads to the formation on the skin of a film-forming active complex with plasticizing effect which, in combination with an extract or a combination of plant extracts with known astringent effect, allows the formulation of an antiperspirant complex with efficacy equal to that of known formulations, containing metal compounds and silicones and synthetic filming agents.

The invention therefore consists of a deodorant composition with antiperspirant efficacy, as indicated in the main claim, in which the plasticizer and astringent active complex is realized through the synergistic combination of Triethyl Citrate with an extract or a combination of plant extracts with known astringent properties that leads to the production of an antiperspirant, whose activity is based exclusively on natural substances, with an efficacy of at least 48 h.

It has been demonstrated that, an extract or a combination of plant extracts with astringent effect, widely used in cosmetics to reduce skin blemishes, acts on sweat pores, reducing the section of the excretory duct and consequently decreasing the flow of sweat, while Triethyl Citrate, creates a light water-repellent film, allowing to have a long time extract contact with the sweat duct. It is thus possible, to carry out a long-lasting antiperspirant action, even up to 48 hours.

The invention therefore makes it possible to overcome the drawbacks of the known technique with a product composed solely of plant extracts, with a verifiable antiperspirant action carried out by mechanisms different from known products containing aluminum hydrochloride and, more generally, aluminum salts or metal compounds, as well as silicones and synthetic filming resins of non-vegetable origin, such as:
- acrylic resins: acrylic acid copolymers of any kind;
- crotonic resins: crotonic acid copolymers with acrylic or other derivatives;
- vinyl resins: homopolymers or copolymers of vinyl acetate with other monomers of any kind;
- urethane resins: polymers of any nature in which the -NH-(CO)-O9 bond is present as a monomer;
- copolymers of acrylic acid and buthylacrylamide;
- copolymers of acrylic acid and acrylamide;
- PVM/MA copolymers, commonly known as Gantrez A-425, ES-225, ES-335, ES-425, ES-435, SP-215, PShield manufactured by Ashland;
- polymers and copolymers of Polyvinyl caprolactame, with or without acrylic acid and other acrylates.

The components according to the invention allow the realization of antiperspirant deodorants both in aerosol format and, with the help of suitable additives, in roll-on, liquid, stick or other convenient format.

The absence of metallic compounds of the formulation also allows the use of dispensers with non-flammable ecological propellants, such as nitrogen and compressed air, as an alternative to the well-known hydrocarbon propellants, such as LPG, without reducing the ability to effectively spray the product throughout its life. The antiperspirant deodorant according to the invention, in water-based aerosol format, is therefore compatible with the use of eco-valves.

In addition, in the preferred formulations, the invention provides advantageously the use of natural pentylene glycol and natural propanediol, avoiding the use of paraben preservatives or other synthetic compounds normally used as preservatives, which can damage the skin microflora.

The formulation does not require the use of ethyl or isopropyl alcohol and any type of flammable solvent.

Finally, Triethyl Citrate makes it possible to produce a product with a eudermic pH between 4.0 and 5.5.

These and other advantages of the invention will be better understood with the disclosure of favorite examples of realization, exposed only by way of example and not limitation.

A first example of formulation of an antiperspirant deodorant, in aerosol form, according to the invention, includes the following components, for which preferred ranges of values are indicated:

| | |
|---|---|
| Deionized water | qs to 100 |
| Triethyl Citrate | 1.0 to 5.0 |
| Emulsifier | 1.0 to 5.0 |
| Natural Pentylene Glycol | 1.0 to 5.0 |
| Commiphora Myrrha Resin Extract | 0.01 to 1.0 |
| Usnea Barbata Extract | 0.01 to 1.0 |
| Propanediol Natural | 1.0 to 5.0 |
| Natural Essence | 1.0 to 3.0 |
| Nitrogen | 0,9 |
| Total | 100 |

It is noted that, in the above example, in the calculation of the total weight of the cosmetic, in addition to the components according to the invention, propellant is included. The example of realization concerns in fact a water-based formulation for aerosol spray in which nitrogen is used as a compressed gas, ideal as a propellant as it is ecological and non-flammable.

As already indicated, the use of ecological propellants is made possible by the fact that the formulation according to the invention, unlike antiperspirants based on aluminum salts or metal compounds, is compatible with the use of eco-valves and dispensing inserts on the market, which allow to spray the product efficiently and stably throughout its life. It is clear, however, that the invention can also find advantageous application in aerosol sprays with traditional flammable propellants or in which a percentage of other gas or LPG is added. For the example formulation, a gravimetric test was conducted in order to verify and claim antiperspirant activity.

The mean armpit perspiration treated with the product and placebo-treated was measured on 20 volunteers at 24 hours and 48 hours after the last daily treatment. The table shows the average sweat collected in grams.

| TIME TEST | PRODUCT | PLACEBO |
|---|---|---|
| T24h | 0,750 | 0,865 |
| T48h | 0,770 | 0,862 |

The mean percentage variation in armpit perspiration treated with the product compared to placebo-treated armpit at 24h and 48h after the last daily treatment was also calculated. In this case there is an average percentage reduction in armpit sweating treated with the formulation according to the invention referred to in example 1, compared to that treated with placebo, after 24h of -13.69% and after 48h of -5.90%.

The production process of the formula shown by way of example involves the simple preparation of an emulsion in water, in which the emulsifier is dissolved in deionized water, then all the other components of the formula are added in sequence, until a homogeneous emulsion is obtained.

This emulsion process does not present particular differences compared to the known processes, used for emulsions of traditional antiperspirant deodorants.

In the example of realization, the components according to the invention are indicated with the INCI name, but advantageously the same components are available with the trade names registered by the different manufacturers.

In a preferred formulation of the antiperspirant deodorant according to the invention as an emulsifier, for example, the TEGO^{®} Solve 90 MB can be used, then a mixture of two polyglyceryl esters such as Polyglyceryl-6 Caprylate and Polyglyceryl-4 Caprate, but it is evident that the same results can be obtained using different polyglyceryl esters, produced by esterification of a polyglycerol and a fatty acid, forming a molecule that contains a hydrophilic head group and a hydrophilic tail group. It, by way of example, can be chosen from Polyglyceryl-3 Polyricinoleate, Polyglyceryl-5 Laurate, Polyglyceryl-6 Disterate, Polyglyceryl-6 Oleate, Polyglyceryl-10 Stearate, Polyglyceryl-10 Oleate, Polyglyceryl-10 Dipalmitate.

Also with regard to astringent compounds it is possible to find and use ready-made mixtures advantageously, such as AKOWELL^{®} FRESH, a mixture of Commiphora Myrrha Resin Extract and Usnea Barbata Extract solubilized in natural Propanediol.

Similar results can also be obtained using other compounds of plant extracts with astringent properties such as, for example, the extracts contained in Laricyl^{®} (composed of Fomes Officinalis (Mushroom) Extract, butylene gycol and PEG-40 Hydrogenated Castor Oil).

A second example of the formulation of an antiperspirant deodorant, in aerosol form, according to the invention, can therefore be based on the use of a known compound and includes the following components, for which preferred ranges of values are indicated:

| | |
|---|---|
| Deionized water | qs to 100 |
| Triethyl Citrate | 3.0 to 5.0 |
| Emulsifierfrom | 2.5 to 3.5 |
| Natural Pentylene Glycol | 2.0 to 3.0 |
| Laricyl^{®} | 2.0 to 5.0 |
| Propanediol Natural | 1.0 to 1.5 |
| Natural Essence | 1,0 to 1,5 |
| Nitrogen | 0,9 |
| Total | 100 |

Below are shown the results of the gravimetric test for this second example showing the average sweat collected in g.

| TIME TEST | PRODUCT | PLACEBO |
|---|---|---|
| T24h | 0,694 | 0,833 |
| T48h | 0,894 | 0,988 |

The mean percentage reduction in armpit sweating treated with the formulation according to the invention referred to in example 2, compared to that treated with placebo, is after 24h of -21.6% and after 48h of -11.4%.

Appreciable results can also be obtained using other compounds of plant extracts with astringent properties such as, for example, ANTI PERSPIRANT NATURAL COMPLEX by Greentech Biotechnologies, composed of Equisetum Arvense Extract, Salvia Officinalis (Sage) Oil.

This compound, used in the percentages indicated in the second example of realization, allowed to obtain an average percentage reduction of sweating of the armpit treated with the formulation, compared to that treated with placebo, after 24 h of -25.9% and after 48h of -14.0%.

The emulsion obtained and according to the invention can be used also in Bag on Valve type dispensers, where the propellant is not in direct contact with the cosmetic preparation, which is inserted into a bag crimped on the can. In addition, the invention is also used for devices without propellant, for example for bottles equipped with spray pumps, in which the product is dispensed by the mechanical effect of the spray pump operated thanks to the pressure on the dispenser.

In these cases the aqueous emulsion according to the invention, without propellant, will be inserted in a known way in the dispensing device.

In general, the invention allows the creation of antiperspirant deodorants also in other cosmetic forms.

By way of example only, an antiperspirant deodorant in "roll on" form according to the invention, can consist of:

| | |
|---|---|
| Aqua | qs to 100 |
| Cetyl alcohol | 4.0 |
| Triethyl Citrate | 1.0 to 5.0 |
| Oleyl erucate | 5.0 to 10.0 |
| Emulsifier | 5.0 to 10.0 |
| Natural Pentylene Glycol | 1.0 to 5.0 |
| Commiphora Myrrha Resin Extract | 0.01 to 1.0 |
| Usnea Barbata Extractfrom | 0.01a 1.0 |
| Propanediol Natural | 1.0 to 5.0 |
| Natural Essence | 0,1 to 3,0 |
| Total | 100 |

As for the creation of "sticks" with deodorizing and antiperspirant action, the formula can provide that water, in part, is replaced by natural waxes.

In this case, an example of the formulation of an antiperspirant according to the invention is as follows:

| | |
|---|---|
| Caprylic/Capric Triglyceride | 20.0 to 30.0 |
| Octyldodecanol | 5.0 to 10.0 |
| Beeswax from | 10.0 to 15.0 |
| Shea Butter Ethyl Esters | 3.0 to 7.0 |
| Triethyl Citrate | 1.0 to 5.0 |
| Commiphora Myrrha Resin Extract | 0.01 to 1.0 |
| Usnea Barbata Extractfrom | 0.01a 1.0 |
| Propanediol Natural | 1.0 to 5.0 |
| Natural Essence | 0,1 to 3,0 |
| Total | 100 |

The examples of realization have been presented only by way of example with reference to the use of a compound of Triethyl Citrate with Commiphora Myrrha Resin Extract and Usnea Barbata Extract, and with other compounds with known astringent properties, which laboratory tests have shown to exert a synergistic film-forming and astringent action that allows to boast an antiperspirant efficacy comparable to that attributed to products known with metal salts.

The same results are however obtainable with the use of other plant extracts which, for the purposes indicated by the invention, have detected astringent properties designed to realize, in combination with Triethyl Citrate, a deodorant with antiperspirant efficacy. The plant extract(s) which can be used in combination in antiperspirant formulations according to the invention may be fluid extracts, dry extracts or soft extracts, obtained from fresh or more commonly dried plant drugs, by appropriate extraction processes; these include the use of appropriate solvents and the use of maceration or percolation, or other suitable processes.

The solvents chosen, depending on the preference of the formulator, in the form of juices, tinctures, hydrolates, can be propylene glycol and/or butylene, glycerin, propanediol, water, supercritical CO2, alcohol, vegetable oils and their mixtures.

In the present invention are preferably chosen liquid extracts that uses as solvents a mixture of propanediol and water.

These plant extracts with astringent activity can be produced, for example, by extraction of the whole plant but also exclusively by extraction from flowers and/or leaves and/or seeds and/or other parts of plants.

With astringent plant extracts, the following INCI names are preferred: Fomes Officinalis (Mushroom) Extract, Diospyros Ebenum Bark Extract, Tilia Platyphyllos Flower Extract, Camellia Sinensis Leaf Extract, Hamamelis Virginiana (Witch Hazel) Leaf Extract, Lavandula Angustifolia (Lavender) Flower Extract, Artemisia Princeps Leaf Extract, , Equisetum Arvense Extract, Sage Officinalis (Sage) Oil, Rosmarinus officinalis (Rosemary) Leaf Extract, Salvia officinalis (Sage) Leaf Extract, Thymus Vulgaris (Thyme) Flower/Leaf Extract, Melissa officinalis Leaf Extract, Salix Alba (Willow) Bark Extract, Rubus idaeus (Raspberry) Fruit Extract, Bambusa Vulgaris Leaf/Stem Extract, Vaccinium Myrtillus Fruit Extract, Betula Alba Leaf Extract, Achillea Millefolium Flower Extract, Urtica Dioica (Nettle) Extract, Vitis Vinifera (Grape) Leaf Extract, Vanilla Planifolia Fruit Extract, Cupressus Sempervirens Fruit Extract, Eugenia Caryophyllus (Clove) Flower Extract, Vaccinium Macrocarpon (Cranberry) Fruit Extract, Fragaria Vesca (Strawberry) Fruit Extract, Glycyrrhiza Glabra (Licorice) Root Extract, Ferula Assa-Foetida Gum Extract, Lamium Album Extract, Citrus Tangerina (Tangerine) Fruit, Paullinia Cupana Fruit Extract, Juniperus Communis Fruit Extract, Saponaria Officinalis Leaf/Root Extract, Foeniculum Vulgare (Fennel) Fruit Extract, Urtica Dioica (Nettle) Extract, Thuja Orientalis Extract, Myrica Cerifera (Bayberry) Bark Extract.

In this invention, Salvia Officinalis (Sage) Leaf Extract, Commiphora Myrrha Resin Extract and Usnea Barbata Extract, Equisetum Arvense Extract, Salvia Officinalis (Sage) Oil, Fomes Officinalis (Mushroom) Extract are preferably used individually or in combinations thereof. It is evident that the invention makes it possible to create an antiperspirant cosmetic product, not only in aerosol format or in the formats indicated in the examples of realization, but in any convenient form, for example mousse or cream, effective and able to overcome the drawbacks of known products, as it consists only of elements of natural origin.

In addition, the possibility of avoiding the use of hydrocarbons, such as propellants that are released into the environment, allows to create a product in non-flammable and eco-sustainable aerosol format.

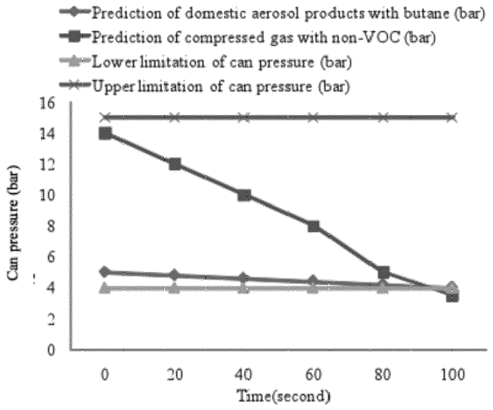

## Claims

1. Cosmetic composition with antiperspirant activity, free of salts of aluminum, zinc, zirconium, magnesium or other metal salts with antiperspirant effect and free of silicones and synthetic filming resins, **characterized by** the fact of being composed of one or more plant extracts with known astringent activity, Triethyl Citrate with plasticizing effect and cosmetically acceptable excipients, in varying percentages.

2. Cosmetic composition with antiperspirant activity according to claim 1 **characterized by** the fact that Triethyl Citrate is present in an amount of at least 0.5% w/w of the composition and preferably from 0.5% to 5% w/w of the composition.

3. Cosmetic composition with antiperspirant activity according to a previous claim **characterized by** the fact that these one or more plant extracts with astringent activity are preferably chosen from Fomes Officinalis (Mushroom) Extract, Diospyros Ebenum Bark Extract, Tilia Platyphyllos Flower Extract, Camellia Sinensis Leaf Extract, Hamamelis Virginiana (Witch Hazel) Leaf Extract, Lavandula Angustifolia (Lavender) Flower Extract, Artemisia Princeps Leaf Extract, Equisetum Arvense Extract, Salvia officinalis (Sage) Oil, Commiphora Myrrha Resin Extract, Usnea Barbata Extract.

4. Cosmetic composition with antiperspirant activity according to a previous claim **characterized by** the fact that these one or more plant extracts with astringent activity are chosen from Rosmarinus Officinalis (Rosemary) Leaf Extract, Salvia Officinalis (Sage) Leaf Extract, Thymus Vulgaris (Thyme) Flower/Leaf Extract, Melissa Officinalis Leaf Extract, Salix Alba (Willow) Bark Extract, Rubus Idaeus (Raspberry) Fruit Extract, Bambusa Vulgaris Leaf/Stem Extract, vaccinium Myrtillus Fruit Extract, Betula Alba Leaf Extract, Achillea Millefolium Flower Extract, Urtica Dioica (Nettle) Extract, Vitis Vinifera (Grape) Leaf Extract, Vanilla Planifolia Fruit Extract, Cupressus Sempervirens Fruit Extract, Eugenia Caryophyllus (Clove) Flower Extract, Vaccinium Macrocarpon (Cranberry) Fruit Extract, Fragaria Vesca (Strawberry) Fruit Extract, Glycyrrhiza Glabra (Licorice) Root Extract, Ferula Assa-Foetida Gum Extract, Lamium Album Extract, Citrus Tangerina (Tangerine) Fruit, Paullinia Cupana Fruit Extract, Juniperus Communis Fruit Extract, Saponaria Officinalis Leaf/Root Extract, Foeniculum Vulgare (Fennel) Fruit Extract, Urtica Dioica (Nettle) Extract, Thuja Orientalis Extract, Myrica Cerifera (Bayberry) Bark Extract.

5. Cosmetic composition with antiperspirant activity according to claims 1 or 2 **characterized by** the fact that preferably this combination of plant extracts consists of Commiphora Myrrha Resin Extract and Usnea Barbata Extract.

6. Cosmetic composition with antiperspirant activity according to the previous claim **characterized by** the fact that Commiphora Myrrha Resin Extract and Usnea Barbata Extract are present in quantities of at least 0.01% w/w of the composition.

7. Cosmetic composition with antiperspirant activity according to one or more previous claims **characterized by** the fact that preferably said combination of plant extracts is composed of Equisetum Arvense Extract, Salvia Officinalis (Sage) Oil.

8. Cosmetic composition with antiperspirant activity according to the previous claim **characterized by** the fact that Equisetum Arvense Extract, Salvia Officinalis (Sage) Oil are present in quantities of at least 0.01% w/w of the composition.

9. Cosmetic composition with antiperspirant activity according to one or more previous claims **characterized by** the fact that these one or more plant extracts are present in an amount of at least 0.02% w/w of the composition.

10. Cosmetic composition with antiperspirant activity according to one or more previous claims whose main solvent is deionized water in percentages ranging from 99% by weight to 0%, preferably from 85% to 20% or oil or natural wax or ethanol.

11. Cosmetic composition with antiperspirant activity according to one or more of the above claims **characterized by** the fact that these cosmetically acceptable excipients include natural pentylene glycol and/or natural propanediol in varying percentages.

12. Cosmetic composition with antiperspirant activity according to one or more previous claims **characterized by** the fact of including Nitrogen as propellant for aerosol sprays, in varying percentages, or compressed gas such as carbon dioxide or compressed air.
